# EUROPEAN PATENT SPECIFICATION

(11) **EP 1 129 711 B1**
(45) Date of publication and mention of the grant of the patent: **28.01.2004**
(21) Application number: 01200471.9
(22) Date of filing: 12.02.2001
(51) Int. Cl.: A61K 31/20, A61K 35/78, A61P 3/10, A61P 17/02, A61P 27/12, A61P 27/02, A61P 13/12, A61P 25/02, A61P 9/12

(54) **Pinolenic acid against diabetes**
Pinolensäure gegen Diabetes
L'acide pinolénique contre le diabète

(30) Priority: 24.02.2000 EP 00301440
(43) Date of publication of application: 05.09.2001
(73) Proprietor: UNILEVER N.V., 3013 AL Rotterdam (NL); UNILEVER PLC, London EC4P 4BQ (GB)
(72) Inventor: Brown, Louise, Sharnbrook, Bedford, MK44 1LQ (GB); Cain, Frederick William, 1521 AZ Wormerveer (NL); Green, Martin Richard, Sharnbrook, Bedford, MK44 1LQ (GB); Rogers, Julia Sarah, Sharnbrook, Bedford, MK44 1LQ (GB)
(74) Representative: Wurfbain, Gilles L.

(56) References cited:
- EP-A- 1 013 278
- EP-A- 1 088 552
- DE-A- 19 503 993
- FR-A- 2 756 465
- US-A- 5 902 726
- PATENT ABSTRACTS OF JAPAN vol. 010, no. 219 (C-363), 31 July 1986 (1986-07-31) & JP 61 058536 A (NIPPON OIL & FATS CO LTD;OTHERS: 01), 25 March 1986 (1986-03-25)

## Description

Insulin resistance is a classical feature of type II diabetes and is an established risk factor for stroke, hypertension, and coronary artery disease. Although pharmaceutical compositions are currently available to prevent and/or treat these diseases, these compositions comprise of synthetic compounds, which are unsuitable for use in food. Therefore we investigated whether we could identify compositions, that would be beneficial in the prevention and / or treatment of the above diseases and that could be used in food products or food supplements, in which the active components consist of natural and / or food grade compounds that do not display the negative effects of the pharmaceuticals. Moreover these compounds should have no detrimental effect on the food performance with regard to taste and texture.

Peroxisome proliferator activated receptors (=PPARs) represent a subset of the nuclear receptor superfamily. To date, 3 isotypes (alpha, beta and gamma) have been identified. These transcription factors bind DNA as heterodimeric complexes and are activated in a ligand-dependent manner. Numerous natural and synthetic agents have already been shown to act as PPAR activators. PPAR-alpha (α) and - gamma (γ) were initially characterised as important regulators of lipid metabolism. However since then other roles for these receptors have been identified as well. These include anti-inflammatory, anticancer, insulin-sensitising, epidermal effects and sebocyte differentiation.

We established an easy and rapid screening assay which can be used to identify PPARγ activators. This assay is based on that described by Kliewer et al (Nature 358 771-774 1992). In brief, cos-7 cells (ECACC No. 87021302) were seeded in 24-well plates at a density of 0.325x10⁵ cells/well. Cells were grown overnight at 37°C/5% CO₂ in DMEM containing 10% FCS, 2mM L-glutamine, 100iu/ml penicillin and 100g/ml streptomycin. Cells were washed with transfection media (DMEM containing 2mM L-glutamine) then transiently transfected with 4 plasmids: a PPAR-responsive firefly luciferase reporter gene (pPPRE3TK-luc); mammalian expression plasmids (pcDNA3/hPPARγ1 and pRSV/hRXRα) containing human PPARγ1 and RXRα cDNAs respectively and a control plasmid (pRLTK, Promega) which constitutatively expresses the renilla luciferase gene. Transfection was performed usihg Lipofectamine (Gibco Brl) as directed by the manufacturers. Transfected cells were incubated for 6h at 37°C/5% CO₂ and then for a further 46h in the presence or absence of ligand. After 46h cell lysates were prepared and the level of firefly and renilla luciferase determined using the Dual luciferase assay system (Promega) and a MLX microtitre plate luminometer (Dynex). Levels of firefly luciferase (normalised against the renilla luciferase control) provide a measure of reporter gene activity which in turn reflects the level of PPARγ activation. The activity of our natural actives are evaluated and compared with synthetic drugs such as the thiazolidinedione derivatives, which are known to be selective PPAR-gamma ligands and are used in the treatment of insulin-resistance / type II diabetes.

The thiazolidinediones have been shown to reduce elevated plasma glucose, triglycerides, and insulin concentrations in animals with hyperinsulinemia. They can also increase plasma high-density lipoprotein cholesterol concentrations, and have a beneficial effect in lowering blood pressure (Nolan JJ et al. N Engl J Med. 1994; 331: 1188-1193). The use of these drugs has also proved beneficial in the treatment of psoriasis (Pershadsingh et al., Arch. Derm. Res. 134: (10) 1304-1305 1998). According to Fr. 2.756.465 pinolenic acid (from pine nut oil) has a blood lipid lowering effect.

DE 19 503 993 discloses the use of an omega-3 fatty acid for treatment of diabetes mellitus.

To confirm that our natural PPAR-γ agonists are able to offer anti-diabetic benefits their ability to enhance glucose uptake in human skeletal muscles has been assessed. This was done using an *in vitro* cell-based assay. Human skeletal muscle cells (SMCs) were obtained from TCS 2000 Ltd. Cells were seeded into 12-well plates at a density of 3x10⁴ cells/well and then grown and differentiated as instructed by TCS. Differentiated human SMCs were incubated, for 32 hours at 37°C/5% CO₂, in differentiation media (supplied by TCS) supplemented with an appropriate amount of agent or vehicle. Media was then changed to serum free media (MEM-alpha media, 2mM glutamine, 100iu/ml penicillin and 100µg/ml streptomycin), supplemented as before, and the cells incubated for a further 16 hours. Cells were then washed 3x with Krebs Ringer Phosphate (KRP) buffer [136mM NaCl, 4.7mM KCl, 1.25mM CaCl₂, 1.25mM MgSO₄, 10mM sodium phosphate buffer, pH 7.4] and incubated in KRP (0.5ml/well) for 30 minutes at 37°C/5% CO₂. After 30 minutes the assay was initiated by addition of 25µl KRP containing 5µCi [³H]2-deoxyglucose and 1mM deoxyglucose. After 10 minutes the reaction was terminated by washing the cells 3x with ice-cold PBS. Cells were then solublised with 125µl/well of 1% Triton X-100 for 20 minutes at 37°C. Radioactive incorporation was measured by scintillation counting. 100µl was analysed for each sample. The ability to enhance glucose uptake indicates that an agent has a hypoglycaemic effect and therefore will be beneficial for lowering the high blood glucose levels associated with diabetes and insulin resistance.

The insulin-sensitising properties of PPAR-γ ligands offers attractive commercial opportunities for the use of these ligands within the food market. It is important that the ligands should be food grade, so natural compounds, present within food ingredients would be a very desirable source. So far a range of PPARγ ligands have been identified, these include synthetic compounds such as the thiazolidinedione derivatives (a synthetic drug). However these compounds cannot be used in foods. Food grade PPARγ activators thus would constitute a major advantage as a nutraceutical for the treatment of insulin resistance and related disorders. The incorporation into food products / supplements being beneficial in the dietary control of these conditions.

The above investigations resulted in the finding of a novel use of a known composition. It was found that a fatty acid moiety in the form of a free acid, an alkyl ester with 1 to 20 C-atoms in the alkyl group thereof, or a mono-di-or triglyceride thereof can be used for the preparation of a food product or a food supplement with the ability to improve the insulin action and to prevent and/or treat insulin resistance and related disorders thereof in humans, if pinolenic acid is applied as the fatty acid moiety. It was found that this fatty acid moiety as defined above can be incorporated into supplements or food products without deteriorating the product performance.

The amount of the fatty acid moiety formulated in the food product, should be such that the consumption of a daily portion gives sufficient intake of the fatty acid moiety to be effective in the prevention / treatment of the above disorders. This can be achieved if the fatty acid moiety is applied in the food product in an amount of 0.1 to 10 wt% on total food product.

It was found that the food products and food supplements comprising the above fatty acid moieties can be applied effectively for the prevention and/or treatment of insulin resistance and/or type II diabetes and/to reduce the increased risk of stroke and/or hypertension and/or coronary artery diseases and/or ulcers and/or cataracts and/or diabetic complications such as nephropathy and/or retinopathy and/or diabetic neuropathies in humans. Further it was, found that these products, comprising the fatty acid moieties according to the invention can also be used for the treatment of gestational diabetes and/or glucose intolerance associated with pregnancy.

Our invention concerns the use of edible compositions containing pinolenic acid for the preparation of functional food compositions or food supplements (capsules, tablets, emulsions, oils, powders). The invention further relates to a use for preventing and/or treating insulin resistance and/or disorders related thereto. This is achieved, by administering to a human suffering from such resistance or a related disorder, an effective amount of pinolenic acid either in the form of a free acid or in the form of an alkyl ester with 1 to 20 C-atoms or in the form of a mono-di or triglyceride via either a supplement or a food composition comprising sufficient amounts of the free acid or alkyl ester or glyceride to provide the required effective amount by eating a daily portion of the food.

Pinolenic acid is present in amounts of up to 26 wt% in pine nut oil. Therefore this oil can be used as source for our pinolenic acid. However it is also possible to apply a concentrate of pinolenic acid. Concentrates with more than 28 wt% pinolenic acid are disclosed in our co-pending EP application 99307752.8. The pinolenic acid compounds can be used as such, or as a blend with fats, eg. the blends disclosed in the above EP application. A very efficient way of administering the pinolenic acid is by incorporating it, in particular as a glyceride, in a food.
It is emphasized here that pine needle oil (e.g. as applied in EP 1.013.278) does not contain pinolenic acid.

Food products typically are selected from the group consisting of: margarines, spreads, mayonnaise, dressings, ice cream, creams, confectionery products (in particular fillings), jam, bakery products (cakes, puff-pastries, laminated products etc.), cream alternatives, soups, drinks, beverages, chocolate, sauces and speciality foods for diabetics with low sugar or low carbohydrate contents.

### EXAMPLES

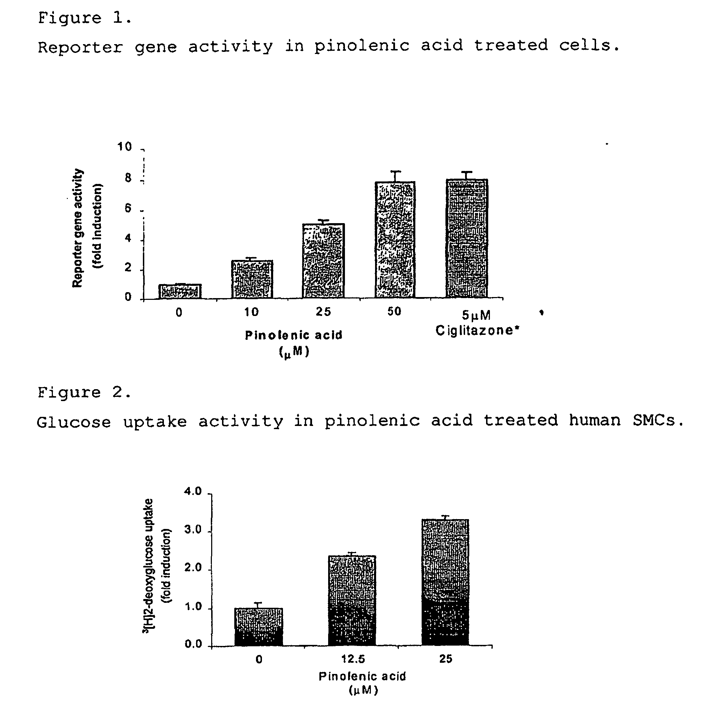

The reporter gene assay was performed as described in the text, the activity of pinolenic acid being compared with that of ciglitazone (a Thiazolidinedione derivative). As demonstrated in Figure 1, Pinolenic acid was a good PPARγ activator.
To confirm the hypoglycaemic effects of Pinolenic acid the ability to modulate glucose uptake in human SMCs has been assessed using the method described in the text. As shown in Figure 2, Pinolenic acid can increase glucose uptake by over 3-fold at micromolar concentrations.

## Claims

1. Use of a fatty acid moiety in the form of a free acid, an alkyl ester with 1 to 20 C-atoms in the alkyl group thereof, or a mono-di-or triglyceride thereof for the preparation of a food product or a food supplement with the ability to prevent and/or treat human diseases or disorders wherein pinolenic acid is applied in the food product or food supplement as the fatty acid moiety to improve insulin action and to prevent and/or treat insulin resistance and/or type II diabetes and/or hypertension and/or ulcers and/or cataracts and/or diabetic complications such as nephropathy and/or retinopathy and/or diabetic neuropathies in humans.

2. Use of the fatty acid moiety according to claim 1 wherein this fatty acid moiety is used for the preparation of a food product selected from the group consisting of: margarines, spreads, mayonnaise, dressings, ice cream, creams, confectionery products (in particular fillings), bakery products (cakes, puff-pastries, laminated products etc.), cream alternatives, soups, drinks, jam, cakes, beverages, chocolate, sauces, and speciality food for diabetics with low sugar or low carbohydrate content.

3. Use of the fatty acid moiety according to claims 1 or 2 wherein the fatty acid moiety is applied in an amount of 0.1 to 10 wt% on total food product.

4. Use of the fatty acid moiety according to claims 1 to 3 wherein the fatty acid moiety is applied for the preparation of a food product or food supplement with the ability to treat gestational diabetes and/or glucose intolerance associated with pregnancy, in humans.

5. Use according to any one of claims 1 to 4, wherein a daily portion of the food product is eaten by a human to improve insulin action and to prevent and/or treat insulin resistance and/or type II diabetes and/or hypertension and/or ulcers and/or cataracts and/or diabetic complications such as nephropathy and/or retinopathy and/or diabetic neuropathies in humans.

## Patentansprüche

1. Verwendung einer Fettsäure-Komponente in Form einer freien Säure, eines Alkylesters mit 1 bis 20 C-Atomen in der Alkylgruppe desselben oder eines Mono-, Di- oder Triglycerids derselben zur Herstellung eines Lebensmittelprodukts oder einer Nahrungsergänzung mit der Fähigkeit, Krankheiten oder Störungen bei Menschen zu verhindern und/oder zu behandeln, wobei Pinolensäure im Lebensmittelprodukt oder in der Nahrungsergänzung als Fettsäurekomponente angewendet wird, um die Insulinwirkung zu verbessern und Insulinresistenz und/oder Typ-II-Diabetes und/oder Bluthochdruck und/oder Geschwüre und/oder Kataracte und/oder Diabetes-Komplikationen, wie z.B. Nephropathie und/oder Retinopathie und/oder diabetische Neuropathien, bei Menschen zu verhindern und/oder zu behandeln.

2. Verwendung einer Fettsäure-Komponente nach Anspruch 1, wobei diese Fettsäurekomponente zur Herstellung eines Lebensmittelproduktes, ausgewählt aus der Gruppe bestehend aus Margarinen, Aufstrichen, Mayonnaise, Dressings, Speiseeis, Sahne (Cremes), Konditoreiwaren (insbesondere Füllungen), Bäckereiprodukten (Kuchen, Blätterteigprodukte, Schichtprodukte, usw.), Sahnealternativen, Suppen, Drinks, Marmeladen, Kuchen, Getränken, Schokolade, Soßen und Spezialitätenlebensmittel für Diabetiker mit niedrigem Zucker- oder niedrigem Kohlenhydrat-Gehalt, verwendet wird.

3. Verwendung der Fettsäure-Komponente nach Anspruch 1 oder Anspruch 2, wobei die Fettsäure-Komponente in einer Menge von 0,1 bis 10 Gew.-% bezogen auf das Gesamtlebensmittelprodukt angewendet wird.

4. Verwendung der Fettsäure-Komponente nach den Ansprüchen 1 bis 3, wobei die Fettsäurekomponente zur Herstellung eines Lebensmittelproduktes oder einer Nahrungsergänzung mit der Fähigkeit, Schwangerschaftsdiabetes und/oder Glucoseintoleranz, verbunden mit einer Schwangerschaft, bei Menschen zu behandeln, angewendet wird.

5. Verwendung nach einem der Ansprüche 1 bis 4, wobei eine tägliche Portion des Lebensmittelproduktes von einem Menschen gegessen wird, um die Insulinwirkung zu verbessern und Insulinresistenz und/oder Typ-II-Diabetes und/oder Bluthochdruck und/oder Geschwüre und/oder Kataracte und/oder Diabetes-Komplikationen, wie z.B. Nephropathie und/oder Retinopathie und/oder diabetische Neuropathien, bei Menschen zu verhindern und/oder zu behandeln.

## Revendications

1. Utilisation d'un fragment d'acide gras sous forme d'acide libre, d'un ester alkylique ayant de 1 à 20 atomes de carbone dans son groupe alkyle, ou de mono-, di- ou triglycéride de celui-ci pour la préparation d'un produit alimentaire ou d'un aliment complémentaire capable de prévenir et/ou de traiter des maladies ou troubles humains, dans laquelle l'acide pinolénique est appliqué dans le produit alimentaire ou le complément alimentaire en tant que fragment d'acide gras afin d'améliorer l'action de l'insuline et de prévenir et/ou traiter l'insulinorésistance et/ou le diabète de type II et/ou l'hypertension artérielle et/ou les ulcères et/ou les cataractes et/ou les complications diabétiques telles que la néphropathie et/ou la rétinopathie et/ou les neuropathies diabétiques chez l'homme.

2. Utilisation du fragment d'acide gras selon la revendication 1, dans laquelle ce fragment d'acide gras est utilisé pour la préparation d'un produit alimentaire sélectionné parmi le groupe constitué de : margarines, pâtes à tartiner, mayonnaise, assaisonnements, glaces, crèmes, produits de confiserie (en particulier les garnitures), produits de pâtisserie (gâteaux, feuilletés, produits feuilletés, etc.), variantes de crème, soupes, boissons, confiture, gâteaux, breuvages, chocolat, sauces et aliments spéciaux pour les diabétiques, pauvres en sucres ou en glucides.

3. Utilisation du fragment d'acide gras selon la revendication 1 ou 2, dans laquelle le fragment d'acide gras est appliqué en une quantité de 0,1 à 10 % en poids par rapport à la totalité du produit alimentaire.

4. Utilisation du fragment d'acide gras selon la revendication 1 à 3, dans laquelle le fragment d'acide gras est appliqué pour la préparation d'un produit alimentaire ou d'un aliment complémentaire capable de traiter le diabète dû à la grossesse et/ou l'intolérance au glucose associée à la grossesse chez l'être humain.

5. Utilisation selon l'une quelconque des revendications 1 à 4, dans laquelle une portion quotidienne du produit alimentaire est mangée par un homme afin d'améliorer l'action de l'insuline et de prévenir et/ou traiter l'insulinorésistance et/ou le diabète de type II et/ou l'hypertension artérielle et/ou les ulcères et/ou les cataractes et/ou les complications diabétiques telles que la néphropathie et/ou la rétinopathie et/ou les neuropathies diabétiques chez l'homme.
